(19) Europäisches Patentamt
European Patent Office
Office européen des brevets

(11) EP 0 880 961 B1

(12) **FASCICULE DE BREVET EUROPEEN**

(45) Date de publication et mention de la délivrance du brevet:
**07.03.2001 Bulletin 2001/10**

(51) Int Cl.[7]: **A61K 7/42**, A61K 7/48

(21) Numéro de dépôt: **98400992.8**

(22) Date de dépôt: **23.04.1998**

(54) **Composition comprenant un dérivé de l'acide cinnamique et un polymère polyaminé**

Ein Zimtsäurederivat und ein Polyamin-Polymer enthaltende Zusammensetzung

Composition comprising a derivative of cinnamic acid and a polyamino-polymer

(84) Etats contractants désignés:
**DE ES FR GB IT**

(30) Priorité: **28.05.1997 FR 9706532**

(43) Date de publication de la demande:
**02.12.1998 Bulletin 1998/49**

(73) Titulaire: **L'OREAL**
**75008 Paris (FR)**

(72) Inventeurs:
- **Boussouira, Boudiaf**
  **75020 Paris (FR)**
- **Candau, Didier**
  **91570 Bievres (FR)**

(74) Mandataire: **Miszputen, Laurent**
**L'OREAL-DPI**
**6 rue Bertrand Sincholle**
**92585 Clichy Cédex (FR)**

(56) Documents cités:
**EP-A- 0 590 538** **FR-A- 2 658 076**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne de nouvelles compositions cosmétiques et/ou dermatologiques (ci-après appelées compositions antisolaires) destinées à la protection de la peau et/ou des cheveux contre les rayonnements UV, en particulier le rayonnement solaire. Plus précisément, elle concerne de nouvelles compositions cosmétiques et/ou dermatologiques présentant une photostabilité améliorée et comprenant, dans un support cosmétiquement et/ou dermatologiquement acceptable, l'association de deux composés particuliers.

L'invention concerne également l'utilisation de ces compositions dans les domaines cosmétique et/ou dermatologique.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination d'UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

Ainsi, dans le but d'assurer une protection de la peau et des cheveux contre l'ensemble du rayonnement UV qui soit la plus complète et la plus efficace possible, on utilise généralement dans la fabrication des compositions antisolaires des associations de filtres actifs dans l'UVA et de filtres actifs dans l'UVB.

A cet égard, une famille de filtres UV-B particulièrement intéressante est actuellement constituée par les dérivés de l'acide cinnamique, et notamment le p-méthoxycinnamate de 2-éthylhexyle, qui présentent en effet un fort pouvoir d'absorption intrinsèque. En outre, d'autres dérivés de l'acide cinnamique ont des propriétés très intéressantes en cosmétique, comme par exemple l'acide caféique, qui est bien connu comme agent dépigmentant.

Or, certains dérivés de l'acide cinnamique présentent l'inconvénient de se dégrader chimiquement dans certaines conditions, notamment lorsqu'ils sont exposés aux UV. Lorsqu'ils subissent une telle dégradation, ces actifs dérivés de l'acide cinnamique perdent leur potentiel d'actif.

On utilise fréquemment dans des compositions anti-UV des dérivés de tocophérol, qui sont connus pour leurs propriétés anti-radicaux libres et qui contribuent par ces propriétés à conserver à la peau sa jeunesse.

Toutefois, la demanderesse a constaté que les dérivés de l'acide cinnamique, en particulier du p-méthoxycinnamate de 2-éthylhexyle, lorsqu'ils sont en présence de dérivés de tocophérol, et sous irradiation UV, se dégradent chimiquement de façon importante. Dans ces conditions, l'association des deux composés ne permet plus une protection solaire large prolongée de la peau et des cheveux.

Une telle réaction de dégradation est particulièrement importante lorsque le dérivé de l'acide cinnamique est associé à un autre filtre organique, en particulier les filtres de la famille du dibenzoylméthane, qui sont des filtres anti-UVA. En particulier l'association de dérives de l'acide cinnamique avec le 4-tert-butyl-4'-méthoxydibenzoyl-méthane est très intéressante. Ces agents filtrants sont très souvent utilisés en association dans les produits pour la peau et les cheveux du fait que leurs spectres d'activité sont complémentaires.

Or, à la suite d'importantes recherches menées dans le domaine de la photoprotection évoqué ci-dessus, la demanderesse a maintenant découvert que l'introduction de certains polymères polyaminés, qui seront définis plus précisément ci-après, dans une composition contenant un dérivé de l'acide cinnamique, en particulier du p-méthoxycinnamate de 2-éthylhexyle, permettait d'améliorer de façon tout à fait remarquable la photostabilité de ce composé au sein de telles compositions, et donc l'efficacité globale de ces compositions.

La présente invention a donc pour objet de nouvelles compositions cosmétiques et/ou dermatologiques comprenant, dans un support cosmétiquement et/ou dermatologiquement acceptable :

a) au moins un dérivé de l'acide cinnamique répondant à la formule (I) suivante :

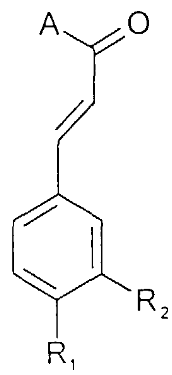

(I)

dans laquelle :
A représente :

- un radical $OR_3$, $R_3$ étant choisi parmi : un atome d'hydrogène, un radical phytyle ou benzyle, une chaîne alkyle en $C_1$-$C_{18}$, linéaire, ramifiée ou cyclique, saturée ou insaturée, un sel de métal alcalin ou alcalino terreux, un ion ammonium,

ou

- un radical $NHR_4$, $R_4$ étant choisi parmi : un atome d'hydrogène, un radical phytyle ou benzyle, une chaîne alkyle en $C_1$-$C_{18}$, linéaire, ramifiée ou cyclique, saturée ou insaturée.

$R_1$ représente un radical choisi parmi : H, OH, alcoxy en $C_1$-$C_6$, préférentiellement méthoxy, une chaîne alkyle en $C_1$-$C_{18}$, linéaire, ramifiée ou cyclique, saturée ou insaturée ;
$R_2$ représente un radical choisi parmi : H, OH, alcoxy en $C_1$-$C_6$, préférentiellement méthoxy, et
b) au moins un polymère polyaminé choisi parmi les familles suivantes :

(A) une polyalkylène polyamine ou un dérivé de polyalkylène polyamine choisi parmi :

(i) les polyalkylène polyamines ;
(ii) les dérivés alkylés de polyalkylène polyamines ;
(iii) les produits d'addition d'acides alkylcarboxyliques sur les polyalkylènepolyamines ;
(iv) les produits d'addition de cétones et d'aldéhydes sur les polyalkylènepolyamines ;
(v) les produits d'addition d'isocyanates et d'isothiocyanates sur les polyalkylènepolyamines ;
(vi) les produits d'addition d'oxyde d'alkylène ou de polymères blocs polyoxyde d'alkylène sur les polyalkylènepolyamines ;
(vii) les dérivés quaternisés de polyalkylène polyamines ;
(viii) les produits d'addition d'une silicone sur les polyalkylènepolyamines ;
(ix) un copolymère d'acide dicarboxylique et de polyalkylène polyamines ;

(B) les polyvinylimidazoles ;
(C) les polyvinylpyridines ;
(D) les produits d'addition de monomères 1-vinylimidazole de formule (1) :

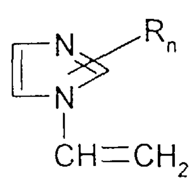

(1)

dans laquelle les radicaux R, identiques ou différents, représentent H, ou un radical alkyle en $C_1$-$C_6$, saturé ou insaturé, linéaire ou cyclique

n est un entier allant de 1 à 3,
avec les polyalkylènepolyamines (A)(i) à (A)(ix) ;
(E) les polymères à base d'acides aminés à chaîne latérale basique ;
(F) les dérivés réticulés des polymères (A)(i) à (A)(ix), (B) (C) (D) et (E) ;.

L'invention a également pour objet de nouvelles compositions cosmétiques et/ou dermatologiques comprenant, dans un support cosmétiquement et/ou dermatologiquement acceptable :

a) au moins un dérivé de l'acide cinnamique répondant à la formule (I) définie ci-dessus ;
b) au moins un polymère polyaminé tel que défini ci-dessus ;
et c) au moins un dérivé répondant à la formule (II) :

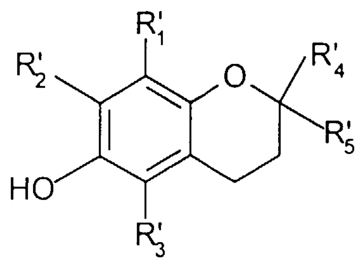

(II)

dans laquelle :

$R'_1$, $R'_2$, $R'_3$, identiques ou différents représentent un radical choisi parmi : H, OH, alkyle en $C_1$-$C_6$, préférentiellement méthyle,
$R'_4$ représente un radical choisi parmi : H, alkyle en $C_1$-$C_6$, préférentiellement méthyle,
$R'_5$ représente un radical choisi parmi : H, alkyle en $C_1$-$C_{18}$,

parmi lesquels on peut citer en particulier les dérivés de tocophérol.

L'invention a en outre pour objet de nouvelles compositions cosmétiques et/ou dermatologiques comprenant, dans un support cosmétiquement et/ou dermatologiquement acceptable :

a) au moins un dérivé de l'acide cinnamique répondant à la formule (I) définie ci-dessus,
b) au moins un polymère polyaminé tel que défini ci-dessus,
et d) au moins un au moins un dérivé de dibenzoylméthane répondant à la formule (III) suivante :

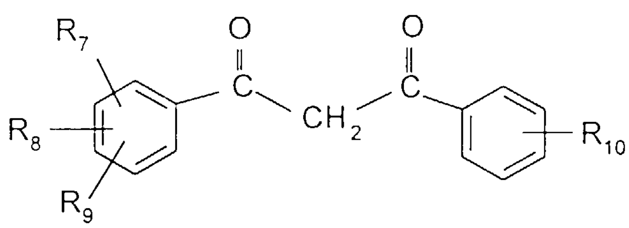

(III)

dans laquelle $R_7$, $R_8$, $R_9$ et $R_{10}$, identiques ou différents, représentent indépendamment l'hydrogène ou un radical hydroxyle ou un radical alkyle linéaire ou ramifié en $C_1$-$C_8$ ou un radical alcoxy linéaire ou ramifié en $C_1$-$C_8$.

Ainsi, selon la présente invention, on peut réaliser des compositions cosmétiques et/ou dermatologiques contenant un dérivé de l'acide cinnamique, en particulier du p-méthoxycinnamate de 2-éthylhexyle, éventuellement en association avec au moins un dérivé de formule (II), en particulier un dérivé de tocophérol ou au moins un dérivé de dibenzoylméthane, compositions dans lesquelles la concentration en dérivé de l'acide cinnamique reste relativement constante même si ces compositions sont soumises à l'action de la lumière.

La présente invention a encore pour objet l'utilisation d'un polymère polyaminé dans, ou pour la fabrication de, compositions cosmétiques et/ou dermatologiques contenant un dérivé de l'acide cinnamique tel que défini ci-dessus (formule (I)), en particulier du p-méthoxycinnamate de 2-éthylhexyle, éventuellement en association avec au moins un dérivé de formule (II) tel que défini ci-dessus et/ou en association avec au moins un dérivé de dibenzoylméthane

tel que défini ci-dessus (formule (III)) dans le but d'améliorer dans ces compositions la stabilité au rayonnement UV (photostabilité) du dérivé de l'acide cinnamique.

La présente invention a également pour objet un procédé pour améliorer la stabilité au rayonnement UV (photostabilité), et donc l'efficacité, d'une composition cosmétique et/ou dermatologique comprenant un dérivé de l'acide cinnamique tel que défini ci-dessus (formule (I)), en particulier du p-méthoxycinnamate de 2-éthylhexyle, et éventuellement un dérivé de formule (II) tel que défini ci-dessus et/ou un dérivé de dibenzoylméthane tel que défini ci-dessus (formule (III)), ce procédé consistant à introduire dans la composition une quantité efficace d'un polymère polyaminé.

Par quantité efficace de polymère polyaminé, on entend une quantité suffisante pour obtenir une amélioration notable et significative de la photostabilité du ou des dérivés de l'acide cinnamique contenus dans la composition. Cette quantité minimale en agent stabilisant à mettre en oeuvre, qui peut varier selon la nature du support cosmétiquement acceptable retenu pour la composition, peut être déterminée sans aucune difficulté au moyen d'un test classique de mesure de photostabilité, tel que celui donné dans les exemples ci-après.

D'autres caractéristiques, aspects et avantages de la présente invention apparaîtront à la lecture de la description détaillée qui va suivre.

Les polymères polyaminés utilisables dans la présente invention peuvent se trouver sous la forme de polymère linéaire, de polymère hyperbranché ou de dendrimère.

Les polymères hyperbranchés sont des constructions moléculaires ayant une structure ramifiée, en général autour d'un coeur. Leur structure est en règle générale dépourvue de symétrie : les unités de base ou monomères ayant servi à la construction du polymère hyperbranché peuvent être de natures différentes et leur répartition est irrégulière. Les branches du polymère peuvent être de natures et de longueurs différentes. Le nombre d'unités de base, ou monomères, peut être différent suivant les différentes ramifications. Tout en étant asymétriques, les polymères hyperbranchés peuvent avoir : une structure extrêmement ramifiée, autour d'un coeur ; des couches ou générations successives de ramifications ; une couche de chaînes terminales.

Les polymères hyperbranchés sont généralement issus de la polycondensation d'un ou plusieurs monomères ABx, A et B étant des groupements réactifs susceptibles de réagir ensemble, x étant un entier supérieur ou égal à 2, mais d'autres procédés de préparation peuvent être envisagés. Les polymères hyperbranchés se caractérisent par leur degré de polymérisation DP = 1-b, b étant le pourcentage de fonctionnalités, non terminales, de B qui n'ont pas réagi avec un groupement A. La condensation étant non systématique, au contraire de la synthèse de dendrimères, le degré de polymérisation est inférieur à 100%. De façon habituelle, par les méthodes de synthèses connues, DP est compris entre 15 et 90%. On peut faire réagir un groupement terminal T sur le polymère hyperbranché pour obtenir une fonctionnalité particulière en extrémité de chaînes.

De tels polymères sont décrits en particulier dans B.I.Voit, Acta Polymer., 46, 87-99 (1995) ; EP-682059 ; WO-9614346 ; WO-9614345 ; WO-9612754.

Plusieurs polymères hyperbranchés peuvent être associés entre eux, par une liaison covalente ou un autre type de liaison, par l'intermédiaire de leurs groupes terminaux. De tels polymères dits pontés ou «bridged» entrent dans la définition des polymères hyperbranchés selon la présente invention.

Les dendrimères sont des polymères et oligomères hautement ramifiés, également connus, ayant une structure chimique bien définie, et on dit que ce sont des polymères hyperbranchés « parfaits ». En règle générale, les dendrimères comprennent un coeur, un nombre déterminé de générations de branches, ou fuseaux, et des groupes terminaux. Les générations de fuseaux sont constituées d'unités structurelles, qui sont identiques pour une même génération de fuseaux et qui peuvent être identiques ou différentes pour des générations de fuseaux différentes. Les générations de fuseaux s'étendent radialement en une progression géométrique à partir du coeur. Les groupes terminaux d'un dendrimère de la $N^{ième}$ génération sont les groupes fonctionnels terminaux des fuseaux de la Nième génération ou génération terminale. De tels polymères sont décrits en particulier dans D.A.Tomalia, A.M.Naylor et W.A. Goddard III, *Angewandte Chemie,* Int.Ed.Engl.29, 138-175 (1990) ; C.J.Hawker et J.M.J.Frechet, *J.Am.Chem.Soc.,* 112, 7638 (1990) ; B.I.Voit, Acta Polymer., 46, 87-99 (1995) ; N.Ardoin et D.Astruc, Bull. Soc. Chim. Fr. 132, 875-909 (1995).

On peut également définir plus particulièrement les dendrimères par la formule (DI) suivante :

$C[A_1B_1(A_2B_2(...(A_{n-1}\ B_{n-1}(A_nB_n(T)r_n)r_{n-1})r_{n-2}...)r_2)r_1]s$ (DI)

dans laquelle :

- C représente le coeur, relié par un nombre s de fonctionnalités à s fuseaux $A_1B_1$ par l'intermédiaire des groupements $A_1$ ;
- s est un nombre entier supérieur ou égal à 1 et inférieur ou égal au nombre de fonctionnalités de C ;
- l'indice i (i=1, 2.....n) est un nombre entier qui désigne la génération de chaque fuseau ;
- $r_i$ (i=1, 2.....n-1) représente le nombre de fonctionnalités du groupement $B_i$ appartenant au fuseau $(A_1B_1)$, $r_i$ étant un entier supérieur ou égal à 2

- pour chaque fuseau ($A_iB_i$) (i=1, 2.....n), le groupement $B_i$ est relié à $r_i$ groupements $A_{i+1}$ d'un fuseau ($A_{i+1}B_{i+1}$) ;
- chaque groupement $A_i$($i \geq 2$) est relié à un seul groupement $B_{i-1}$ du fuseau ($A_{i-1}B_{i-1}$) ;
- le fuseau de $n^{ième}$ génération $A_nB_n$ est chimiquement lié à un nombre $r_n$ de groupes terminaux T, $r_n$ étant un entier supérieur ou égal à zéro.

La définition des dendrimères donnée ci-dessus inclut des molécules à ramifications symétriques ; elle inclut également des molécules à ramification non symétrique, comme par exemple les dendrimères dont les fuseaux sont des groupements lysine, dans lesquels le branchement d'une génération de fuseaux sur la précédente se fait sur les amines $\alpha$ et $\varepsilon$ de la lysine, ce qui conduit à une différence dans la longueur des fuseaux des différentes ramifications.

Les polymères denses en étoiles, ou «dense star polymer», les polymères éclatés en étoile, ou «starburst polymer», les dendrimères en baguette, ou «rod-shaped dendrimer», sont inclus dans la présente définition des dendrimères. Les molécules dénommées arborols et molécules en cascade entrent également dans la définition des dendrimères selon la présente invention.

Plusieurs dendrimères peuvent être associés entre eux, par une liaison covalente ou un autre type de liaison, par l'intermédiaire de leurs groupes terminaux pour donner des entités connues sous le nom de «dendrimères pontés», «agrégats de dendrimères» ou «bridged dendrimer». De telles entités sont incluses dans la définition des dendrimères selon la présente invention.

Des dendrimères peuvent se présenter sous la forme d'un ensemble de molécules de même génération, ensembles dits monodisperses ; ils peuvent également se présenter sous la forme d'ensembles de générations différentes, dits polydisperses. La définition des dendrimères selon la présente invention inclut des ensembles monodisperses aussi bien que polydisperses de dendrimères.

On peut se reporter aux documents suivants dans lesquels sont décrits des dendrimères comportant des groupements fonctionnels amines, le contenu de ces documents étant incorporé par référence dans la présente description : US-4,694,064 ; US-4,631,337 ; WO-A-9502008 ; WO-A-9314147 ; US-4,360,646 ; Proc. Natl. Acad. Sci. USA, 85, 5409-5413 (1988).

Les polymères hyperbranchés et les dendrimères à groupes fonctionnels aminés peuvent également être constitués d'un coeur et de générations d'unités de base, monomères ou fuseaux, de toutes natures, sur lesquels un groupe terminal T porteur d'une fonction amine a été greffé.

On va décrire de façon plus approfondie les polymères polyaminés (A)(i) à (A)(ix), (B), (C), (D), (E) et (F) de l'invention :

(A) (i)les polyalkylène polyamines utilisés préférentiellement selon l'invention sont des polymères contenant de 7 à 20000 motifs de répétition. De préférence, on choisit des polyalkylène polyamines comprenant au moins 5% d'amines tertiaires, avantageusement au moins 10% de fonctions amines tertiaires, et encore plus préférentiellement au moins 20%. Ces polymères peuvent être des homopolymères ou des copolymères, linéaires, branchés ou de structures dendrimériques.

Ces polymères comprennent les motifs répétitifs suivants :

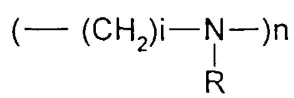

dans lequel :

i représente un entier supérieur ou égal à 2, préférentiellement i=2 ;
n représente un entier
R représente H ou un motif

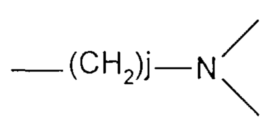

dans lequel j représente un entier supérieur ou égal à 2, préférentiellement j=2 ;

Parmi les produits de la famille des polyalkylène polyamines, appelés aussi polyaziridines, on peut citer en particulier :

La polyéthylèneimine, qui est un polymère hyperbranché bien connu de l'homme du métier : au sujet de la polyéthylèneimine on peut se reporter en particulier aux documents « KIRK-OTHMER ENCYCLOPEDIA OF CHEMICAL TECHNOLOGY », 3ème édition, vol.20, 1982, p214-216 et "Polyéthylèneimine Prospective Application" H.N. Feigenbaum, Cosmetic & Toiletries, 108, 1993, p 73. La polyéthylèneimine est disponible commercialement auprès de la société BASF sous les noms de marque LUPASOL et POLYIMIN ; la polyéthylèneimine est habituellement comprise dans une gamme de poids moléculaire moyen allant de 500 à 2.000.000 ;

On connait aussi des polyéthylèneimines et des polypropylèneimines sous la forme de dendrimères, fabriqués par la société DSM. Les demandes de brevet WO 95/02008 et WO 93/14147, décrivent des polyalkylènepolyamines de la famille des dendrimères ainsi qu'un procédé pour leur préparation.

(A) (ii) les dérivés alkylés de polyalkylène polyamine sont des produits bien connus de l'homme du métier. Ils sont obtenus de façon connue par alkylation, en milieu aqueux ou alcoolique, en présence d'un agent alkylant, de préférence en présence de NaOH, de KOH ou de carbonate, à des températures allant de préférence de 40°C à 130°C. L'agent alkylant peut être choisi par exemple parmi des dérivés sulfate d'alkyle ou halogénure d'alkyle en $C_1$-$C_8$, comme par exemple le diméthylsulfate, le diéthylsulfate, le bromure de butyle, le bromure d'hexyle, le bromure de 2-éthylhexyle, le bromure de n-octyle ou les chlorures correspondants. On peut par exemple se reporter à DE-3743744 qui décrit la préparation de tels produits.

(A) (iii) Les produits d'addition d'acides alkylcarboxyliques sur des polyalkylène polyamines sont des produits connus de l'homme du métier et dont la préparation est décrite par exemple dans les demandes de brevet WO 94/14873 ; WO 94/20681 ; WO 94/12560. L'addition d'acides alkylcarboxyliques sur des polyalkylènepolyamines peut être effectuée en faisant réagir de façon connue un acide, un amide, un ester, un halogénure d'acide sur le polymère polyalkylènepolyamine.

Les produits d'addition d'acides alkylcarboxyliques sur des polyalkylènepolyamines, peuvent être par exemple les produits d'addition d'acides alkylcarboxyliques en $C_2$-$C_{30}$, saturés ou insaturés, linéaires ou ramifiés, sur une polyéthylèneimine. Parmi les acides carboxyliques utilisables, on peut citer par exemple, l'acide acétique, l'acide propionique, l'acide butyrique, l'acide 2-éthylhexanoïque, l'acide benzoïque, l'acide laurique, l'acide myristique, l'acide palmitique, l'acide stéarique, l'acide oléique, l'acide linoléique, l'acide arachidonique, l'acide béhénique, ainsi que les mélanges de corps gras, comme par exemple les mélanges d'esters gras disponibles sous forme de produits naturels, et parmi lesquels on peut citer : l'huile de coco, l'huile de soja, l'huile de lin, l'huile de colza.

(A) (iv) les produits d'addition de cétones et d'aldéhydes sur les polyalkylènepolyamines

(A)(i) peuvent être préparés par des procédés connus de l'homme du métier et conduisent à l'obtention de motifs $\alpha$-hydroxyamine ;

(A) (v) les produits d'addition d'isocyanates et d'isothiocyanates sur les polyalkylénepolyamines (A)(i) peuvent être préparés par des procédés connus de l'homme du métier et conduisent à l'obtention de motifs urée et thio-urée ;

(A) (vi) les produits d'addition d'oxyde d'alkylène ou de polymères blocs polyoxyde d'alkylène sur les polyalkylènepolyamines (A)(i) peuvent être préparés par des procédés connus de l'homme du métier ; on peut se reporter par exemple aux documents EP-541018 et US-4,144,123, dans lesquels sont décrites de telles molécules ; des dérivés de polyéthylèneimine éthoxylés sont disponibles commercialement sous le nom de marque : LUPASOL 61(BASF)

(A) (vii) les dérivés quaternisés de polyalkylène polyamines (A)(i) peuvent être préparés par des procédés connus de l'homme du métier ;

(A) (viii) les produits d'addition d'une silicone sur les polyalkylènepolyamines (A)(i) sont par exemple des polyéthylèneimines greffées par des motifs polydiméthylsiloxane dont la préparation est décrite dans le document US-5,556,616 et commercialisées par la société MAC INTYRE sous le nom de marque MACKAMER PAVS ;

(A) (ix) les copolymères d'acide dicarboxylique et de polyalkylène polyamines (A)(i) peuvent être préparés par polycondensation d'acides dicarboxyliques avec des polyalkylène polyamines.

Parmi les acides dicarboxyliques pouvant être utilisés pour la préparation des polyamidoamines on peut citer les acides dicarboxyliques en $C_2$ à $C_{10}$, comme par exemple l'acide oxalique, l'acide malonique, l'acide itaconique, l'acide succinique, l'acide maléique, l'acide adipique, l'acide glutarique, l'acide sébacique, l'acide téréphtalique, l'acide orthophtalique, ainsi que leurs mélanges.

Les polyalkylène polyamines utilisées pour la préparation des polyamidoamines sont avantageusement choisies parmi celles ayant de 3 à 10 atomes d'azote, comme par exemple la diéthylène tri-amine, la triéthylène tétramine, la dipropylène tri-amine, la tripropylène tétramine, la di-hexaméthylène triamine, l'amino propyléthylène di-amine, la bis-aminopropyléthylène di-amine ainsi que leurs mélanges. On peut également utiliser des polyéthylène imines telles que décrites ci-dessus pour la préparation de polyamidoamines.

De tels composés sont décrits par exemple dans les documents : US 4,144,423 et WO 94/29422.

(B) le terme polyvinylimidazole comprend les homopolymères et les copolymères de polyvinylimidazole (PVI) obtenus par polymérisation radicalaire des monomères de vinylimidazole de structure suivante :

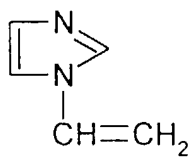

Les copolymères peuvent être par exemple des copolymères de vinylimidazole comportant au moins 5% de motifs vinylimidazole avec des monomères choisis parmi les motifs : vinylpyrrolidinone, acide acrylique et acrylamide. La synthèse de tels composés est bien connue de l'homme du métier ; à ce sujet, on peut en particulier se reporter aux documents : J.Am.Chem.Soc., vol.85, 1962, p.951 ; Polymer Letters Ed., vol.11, 1973, p.465-469 ; Macromolecules, vol.6(2), 1973, p.163-168 ; Ann.N.Y.Acad.Sci., vol.155, 1969, p.431 ; FR-A-1,477,147 ; JP-69 07395 ; J.Macromol. Scien.Chem., vol.A21(2), 1984, p.253.

(C) le terme polyvinylpyridine comprend les homopolymères et les copolymères de vinyl pyridine obtenus par polymérisation radicalaire des monomères de vinyl pyridine (substitués en position 2 ou 4 du noyau pyridine) de structure suivante :

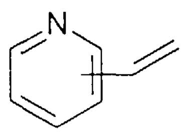

Les copolymères peuvent être par exemple des copolymères de vinylpyridine comportant au moins 5% de motifs vinylpyridine avec des monomères choisis parmi les motifs : vinylpyrrolidinone, acide acrylique et acrylamide.

(D) Les produits d'addition de monomères 1-vinylimidazole répondant à la formule (1) :

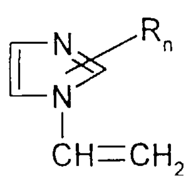

(1)

dans laquelle les radicaux R, identiques ou différents, représentent H, ou un radical alkyle en $C_1$-$C_6$, saturé ou insaturé, linéaire ou cyclique,

n est un entier allant de 1 à 3

avec les polyalkylènepolyamines et leurs dérivés (A)(i) à (A)(ix).

Parmi les dérivés de formule (1) utilisables on peut citer par exemple le 2-méthyl 1-vinyl imidazole, le 2-benzyl 1-vinyl imidazole.

Ces produits sont connus de l'homme du métier: Leur préparation est décrite par exemple dans la demande de brevet WO 94/29422.

(E) Les polymères à base d'acides aminés à chaîne latérale basique sont préférentiellement choisis parmi les protéines et les peptides comprenant au moins 5%, avantageusement au moins 10% d'acides aminés choisis parmi l'histidine, la lysine, l'arginine.

Parmi ces polymères on peut citer par exemple les polylysines, les polyhistidines.

(F) Les dérivés réticulés des polymères (A)(i) à (A)(ix), (B), (C) et (D). Parmi les réticulants utilisables, on peut citer les dérivés halo- hydrin-, glycidyl, aziridino-, isocyanate ; de tels réticulants ainsi que leurs méthodes d'utilisation sont bien connus de l'homme du métier. Parmi les plus connus on peut citer: l'épichlorhydrine, les $\alpha,\omega$-bis-(chlorhydrin) polyalkylène glycoléthers, les $\alpha,\omega$-dichloroalkane comme par exemple le 1,2-dichloroéthane, le 1,2-dichloropropane, le 1,3-dichloropropane, le 1,4-dichlorobutane, et le 1,6-dichlorohexane ; de tels réticulants et leur utilisation pour réticuler des dérivés de polyéthylène imine sont décrits dans WO 94/12560.

De préférence, on choisit dans la mise en oeuvre de la présente invention des polymères polyaminés comprenant au moins 5% d'amines tertiaires, avantageusement au moins 10% de fonctions amines tertiaires, et encore plus préférentiellement au moins 20%.

Selon l'invention, le polymère polyaminé est choisi avantageusement parmi :

(A)

(i) les polyéthylèneimines hyperbranchées,

(ii) les dérivés alkylés de polyéthylèneimine;

(iii) les produits d'addition d'acides alkylcarboxyliques sur la polyéthylèneimine ;

(iv) les produits d'addition de cétones et d'aldéhydes sur la polyéthylèneimine;

(v) les produits d'addition d'isocyanates et d'isothiocyanates sur la polyéthylèneimine ;

(vi) les produits d'addition d'oxyde d'alkylène ou de polymères blocs polyoxyde d'alkylène sur la polyéthylèneimine;

(vii) les dérivés quaternisés de la polyéthylèneimine ;

(viii) les produits d'addition d'une silicone sur les polyéthylèneimine ;

(ix) les copolymères d'acide dicarboxylique et de polyéthylèneimine ;

(B) les polyvinylimidazoles .

Encore plus préférentiellement, le polymère polyaminé est choisi parmi :

(A) (i) les polyéthylèneimines hyperbranchées. De préférence, on choisit des polyéthylèneimines comprenant au moins 5 % d'amines tertiaires, avantageusement au moins 10 % de fonctions amines tertiaires, et encore plus préférentiellement au moins 20 %.

Comme indiqué précédemment, les dérivés de l'acide cinnamique utilisables selon la présente invention sont ceux répondant à la formule (I) ci-dessus.

Selon la présente invention, on peut bien entendu mettre en oeuvre un ou plusieurs dérivés de l'acide cinnamique.

Parmi les dérivés de l'acide cinnamique utilisables selon la présente invention, on peut notamment citer, de manière non limitative : le p-méthoxycinnamate de 2-éthylhexyle, l'acide dihydroxycinnamique également appelé acide caféique, l'acide chlorgénique et les dérivés cafféoylquiniques.

Parmi les dérivés de l'acide cinnamique mentionnés ci-dessus, on préfère tout particulièrement, selon la présente invention, mettre en oeuvre le p-méthoxycinnamate de 2-éthylhexyle, ce filtre répondant donc à la formule développée suivante :

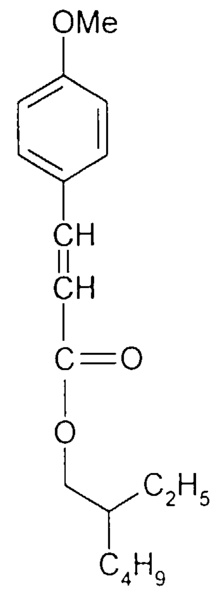

Les dérivés de l'acide cinnamique peuvent être présents dans les compositions de l'invention à une teneur allant de 0,1 % à 10 % en poids, par rapport au poids total de la composition. De préférence, cette teneur va de 0,5 % à 5 %.

Un second composé essentiel des compositions selon l'invention est un composé de la famille des polymères polyaminés.

D'une manière générale, le ou les polymères polyaminés peuvent être présents dans les compositions conformes à l'invention à des teneurs qui sont généralement comprises entre 0,1 % et 10 % en poids, et de préférence à des teneurs comprises entre 0,25 % et 5 % en poids, par rapport au poids total de la composition.

De préférence le polymère polyaminé est introduit dans les compositions selon l'invention sous forme neutralisée.

Ainsi, lorsqu'on ajoute en quantité suffisante un polymère polyaminé à une composition antisolaire contenant un dérivé de l'acide cinnamique, en particulier du p-méthoxycinnamate de 2-éthylhexyle, et un dérivé de formule (II) tel que défini ci-dessus, on observe une augmentation de la stabilité dudit dérivé de l'acide cinnamique à la lumière, et donc une amélioration de l'efficacité de la composition antisolaire au cours du temps.

Avantageusement, le composé de formule (II) est le tocophérol.

Selon cette forme préférée de réalisation de l'invention, le tocophérol est avantageusement présent dans la composition à "l'état libre", c'est-à-dire, sans groupement additionnel et notamment sans groupement formant ester.

De préférence, le tocophérol utilisé est un mélange de tocophérols naturels, en particulier d'α-tocophérol, de β-tocophérol, de γ-tocophérol, et de δ-tocophérol ; ce mélange peut être utilisé notamment dans une huile choisie parmi les huiles végétales, minérales, siliconées, et de préférence végétales.

Comme mélange de tocophérols naturels utilisable selon l'invention, on peut citer celui dissous à 50 % dans l'huile de soja, vendu par la Société BIZEN sous la dénomination D mixed tocopherols. Le D α-tocophérol vendu par la Société HENKEL sous la dénomination Cophérol F1300 ou encore ceux décrits dans le document US-A-4144325 peuvent aussi être utilisés.

Le ou les dérivés de formule (II) sont généralement présents dans les compositions de l'invention à une teneur pouvant aller de 0,5 % à 20 %, de préférence de 1 % à 10 % en poids, par rapport au poids total de la composition.

Les dérivés de dibenzoylméthane répondent à la formule (III) suivante :

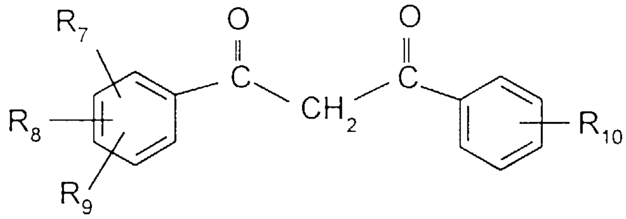
(III)

dans laquelle $R_7$, $R_8$, $R_9$ et $R_{10}$, identiques ou différents, représentent indépendamment l'hydrogène ou un radical hydroxyle ou un radical alkyle linéaire ou ramifié en $C_1$-$C_8$ ou un radical alcoxy linéaire ou ramifié en $C_1$-$C_8$.

Parmi les dérivés du dibenzoylméthane utilisables selon la présente invention, on peut notamment citer, de manière non limitative :

- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- le 4-tert.-butyl-4'-méthoxydibenzoylméthane,
- le 2-méthyl-5-isopropyl-4'-méthoxydibenzoylméthane,
- le 2-méthyl-5-tert.-butyl-4'-méthoxydibenzoylméthane,
- le 2,4-diméthyl-4'-méthoxydibenzoylméthane,
- le 2,6-diméthyl-4-tert.-butyl-4'-méthoxydibenzoylméthane,
- le 4,4'-diméthoxydibenzoylméthane.

Ces produits sont déjà bien connus et sont décrits notamment dans les documents FR-A-2 326 405, FR-A-2 440 933 et EP-A- 0 114 607 précités.

Parmi les dérivés du dibenzoylméthane mentionnés ci-dessus, on préfère tout particulièrement, selon la présente invention, mettre en oeuvre le 4-ter-butyl 4'-méthoxy dibenzoylméthane, ce filtre répondant donc à la formule développée suivante :

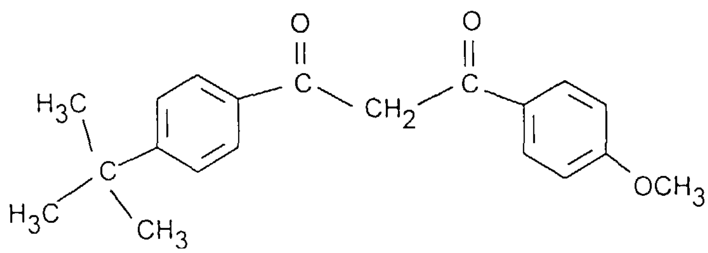

Un autre dérivé du dibenzoylméthane préféré selon la présente invention est le 4-isopropyl-dibenzoylméthane, filtre répondant à la formule développée suivante :

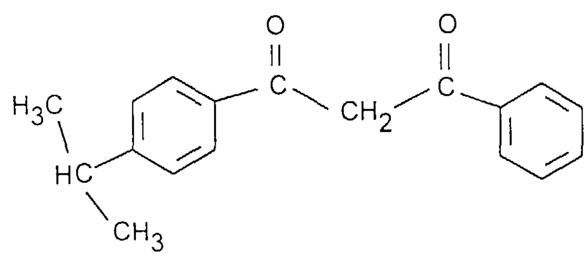

Les dérivés de dibenzoylméthane peuvent être présents dans les compositions de l'invention à une teneur allant de 0,2 % à 15 % en poids, par rapport au poids total de la composition. De préférence, cette teneur va de 0,2 % à 10 %.

Ainsi, lorsqu'on ajoute en quantité suffisante un polymère polyaminé à une composition antisolaire contenant un dérivé de dibenzoylméthane, en particulier du 4-tert-butyl-4'-méthoxydibenzoylméthane, et un dérivé de l'acide cinnamique, on observe une augmentation de la stabilité du dérivé de l'acide cinnamique à la lumière, et donc une amélioration de l'efficacité de la composition antisolaire au cours du temps

Les compositions cosmétiques et/ou dermatologiques visées par la présente invention peuvent bien entendu contenir un ou plusieurs filtres solaires complémentaires actifs dans l'UVA et/ou l'UVB (absorbeurs), hydrophiles ou lipophiles, autres bien sûr que les filtres mentionnés ci-avant. Ces filtres complémentaires peuvent être notamment

choisis parmi les dérivés salicyliques, les dérivés du benzylidène camphre, les dérivés de benzimidazole, les dérivés de triazine, les dérivés de la benzophénone, les dérivés de β, β'-diphénylacrylate, les dérivés de l'acide p-aminobenzoïque, les polymères filtres et silicones filtres décrits dans la demande WO-93/04665. D'autres exemples de filtres organiques sont donnés dans la demande de brevet EP-A 0 487 404.

Les compositions selon l'invention peuvent également contenir des agents de bronzage et/ou de brunissage artificiel de la peau (agents autobronzants), tels que par exemple de la dihydroxyacétone (DHA).

Les compositions cosmétiques et/ou dermatologiques selon l'invention peuvent encore contenir des pigments ou bien encore des nanopigments (taille moyenne des particules primaires : généralement entre 5 nm et 100 nm, de préférence entre 10 et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflexion et/ou diffusion) du rayonnement UV. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A- 0518772 et EP-A- 0518773.

Les compositions conformes à la présente invention peuvent comprendre en outre des adjuvants cosmétiques classiques notamment choisis parmi les corps gras, les solvants organiques, les épaississants ioniques ou non ioniques, les adoucissants, les antioxydants, les agents anti radicaux libres, les opacifiants, les stabilisants, les émollients, les silicones, les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé dans le domaine cosmétique et/ou dermatologique, en particulier pour la fabrication de compositions antisolaires sous forme d'émulsions.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (vaseline); végétales (huile d'amande douce, de macadamia, de pépin de cassis, de jojoba) ; synthétiques comme le perhydrosqualène, les alcools, les acides ou les esters gras (comme le benzoate d'alcools en $C_{12}$-$C_{15}$ vendu sous la dénomination commerciale «Finsolv TN» par la société Finetex, le palmitate d'octyle, le lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthyl cellulose.

Bien entendu, l'homme de l'art veillera à choisir le ou les éventuels composés complémentaires cités ci-dessus (en particulier les filtres complémentaires) et/ou leurs quantités de manière telle que les propriétés avantageuses attachées intrinsèquement à l'association ternaire conforme à l'invention ne soient pas, ou substantiellement pas, altérées par la ou les adjonctions envisagées et de manière telle que les compositions de l'invention présentent de bonnes propriétés cosmétiques.

Les compositions selon l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, ou sous la forme d'un gel ou d'un gel crème, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2315991 et FR 2416008).

La composition cosmétique et/ou dermatologique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95 % en poids, de préférence de 70 à 90 % en poids, par rapport à l'ensemble de la formulation, la phase huileuse (comprenant notamment les filtres lipophiles) de 5 à 50 % en poids, de préférence de 10 à 30 % en poids, par rapport à l'ensemble de la formulation, et le ou les (co)émulsionnant(s) de 0,5 à 20 % en poids, de préférence de 2 à 10 % en poids, par rapport à l'ensemble de la formulation.

Un exemple concret, mais nullement limitatif, illustrant l'invention, va maintenant être donné.

## ESSAIS :

Dans les essais décrits ci-dessous, nous avons utilisé la polyéthylèneimine (PEI) de poids moléculaire 700 commercialisée par la société Aldrich. Les pourcentages indiqués dans la définition des formules sont des pourcentages en poids de constituant par rapport au poids total de la formule.

### Essais 1: Test EX-VIVO d'inhibition par la polyéthylèneimine de la photoperoxydation du p-méthoxycinnamate de 2-éthylhexyle induite par les tocophérols

On applique sur un filtre circulaire de 17cm$^2$ un film mince de composition témoin (formule A : p-méthoxycinnamate de 2-éthylhexyle + Vitamine E) à raison d'environ 3 mg/cm$^2$. On applique sur un second filtre la composition contenant la polyéthylènimine (produit B : p-méthoxycinnamate de 2-éthylhexyle + Vitamine E + PEI). Les filtres sont ensuite irradiés sous 20 joules UVA/cm2 à l'aide d'un appareil Biotronic 360.

Le p-méthoxycinnamate de 2-éthylhexyle et ses peroxydes sont extraits des filtres, à l'aide de 5 ml d'éthanol, en vue du dosage des peroxydes de la formule.

Les résultats sont exprimés en inhibition de la péroxydation du p-méthoxycinnamate de 2-éthylhexyle :

$$\% \text{ inhibition} = \frac{\text{ROOH(formuleA)} - \text{ROOH(formuleB)}}{\text{ROOH(formuleA)}} \times 100$$

ROOH représente la quantité de péroxydes de p-méthoxycinnamate de 2-éthylhexyle dans la composition (picomoles de péroxydes en équivalent $H_20_2$ par mg de produit).

Les compositions des formules A et B sont données dans le tableau ci-dessous :

| Nom CTFA | Formule A | Formule B |
|---|---|---|
| Cetyl alcohol | 5% | 5% |
| Glyceryl stearate | 3% | 3% |
| P.E.G. 50 stearate | 3% | 3% |
| Mineral oil | 18.5% | 18.5% |
| Caprylic/Capric triglycerides | 3% | 3% |
| p-méthoxycinnamate de 2-éthylhexyle | 0.5% | 0.5% |
| $\alpha$ tocopherol | 2% | 2% |
| Water | QSP 100% | QSP 100% |
| Polyéthylènimine | 0% | 1% |

On obtient les résultats suivants :

| Formules | Péroxydes en pmoles/mg |
|---|---|
| Formule A | 2214 |
| Formule B | 332 |

Soit une inhibition de 85% de la photopéroxydation du p-méthoxycinnamate de 2-éthylhexyle de la formule B par rapport à la formule A.

**ESSAI 2 : Test de photostabilisation du p-méthoxycinnamate de 2-éthylhexyle en présence de 4-tert-butyl-4'-méthoxy-dibenzoylméthane par la polyéthylèneimine sous UVA**

Des films de formule anti-solaire sont préparés par étalement manuel de la formule à raison de 2mg/cm$^2$ sur un support en polyméthacrylate de méthyle dépoli (PMMA) ou de verre dépoli.

Les échantillons ainsi préparés sont ensuite exposés pendant 2H30 ou 4H30, au rayonnement d'un Sun-test HERAEUS (source: Arc long Xénon 1,8 kW), dans une enceinte dont la température est régulée aux environs de 35 à 40°C, afin de simuler une irradiation UV naturelle.
Cette exposition correspond à environ 30 J/cm$^2$ UVA ( 2H30) ou 54 J/cm$^2$ UVA (4H30).

Après exposition, les filtres UV sont extraits par 63 ml d'éthanol par échantillon. Les solutions obtenues sont analysées par Spectrophotométrie. On mesure la densité optique de cette solution au $\lambda$max du filtre.
Parallèlement, on extrait et on analyse selon le même protocole les filtres d'un échantillon de formule témoin (même composition) appliqué sur le support de PMMA dépoli ou de verre dépoli, mais n'ayant pas subi d'irradiation UV .

Le taux de filtres résiduels après irradiation est donné, pour chacun des filtres de la formule, par le rapport de la densité optique dans l'échantillon irradié à sa densité optique dans l'échantillon non irradié (échantillon témoin).

La composition des formules testées est la suivante :

| | A' | B' | C' | D' | E' |
|---|---|---|---|---|---|
| -Stéarate de polyéthylène glycol (40 OE) (commercialisé par la société ICI sous le nom de marque Myrj 52) | 2 | 2 | 2 | 2 | 2 |
| -Tristéarate de sorbitane (commercialisé par la société ICI sous le nom de marque Span 65) | 0.9 | 0.9 | 0.9 | 0.9 | 0.9 |
| -Alcool cétylique | 4 | 4 | 4 | 4 | 4 |
| -Stéarate de glycerol codex (STEARINERIE DUBOIS) | 3.3 | 3.3 | 3.3 | 3.3 | 3.3 |
| -Benzoate d'alcools $C_{12}/C_{15}$ (commercialisé par la société WITCO sous le nom de marque Witconol TN) | 10 | 10 | 10 | 10 | 10 |
| -Huile de vaseline | 5 | 5 | 5 | 5 | 5 |
| - 4-tert-butyl-4'-méthoxy-dibenzoylméthane | 2 | / | 2 | 2 | 2 |
| - p-méthoxycinnamate de 2-éthylhexyle | / | 5 | 5 | 5 | 5 |
| -Polyéthylènimine | / | / | / | 4 | 8 |
| -Acide chlorhydrique | / | / | / | qs pH:7 | qs pH:7 |
| -Glycérine | 6 | 6 | 6 | 6 | 6 |
| -EDTA | 0.1 | 0.1 | 0.1 | 0.1 | 0.1 |
| -Conservateurs | qs | qs | qs | qs | qs |
| -Eau déminéralisée qsp | 100 g | 100 g | 100 g | 100 g | 100 g |

On irradie sous 30 J/cm$^2$. On obtient le résultat suivant :

| | % filtre résiduel (4-tert-butyl-4'-méthoxy-dibenzoylméthane) mesuré à 358 nm | % filtre résiduel (p-méthoxy-cinnamate de 2-éthylhexyle) mesuré à 310 nm |
|---|---|---|
| formule A' | 8 ± 1 | - |
| formule B' | - | 61 ± 5 |
| formule C' | 11 ± 3 | 26 ± 3 |
| formule D' | 19 ± 2 | 35 ± 2 |
| formule E' | 27 ± 3 | 43 ± 3 |

**Revendications**

1. Composition cosmétique et/ou dermatologique comprenant, dans un support cosmétiquement et/ou dermatologiquement acceptable :

a) au moins un dérivé de l'acide cinnamique répondant à la formule (I) suivante :

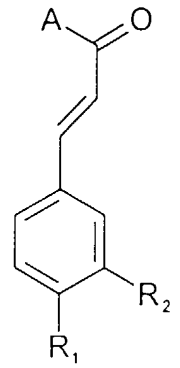

(I)

dans laquelle :
A représente :

- un radical $OR_3$, $R_3$ étant choisi parmi : un atome d'hydrogène, un radical phytyle ou benzyle, une chaîne alkyle en $C_1$-$C_{18}$, linéaire, ramifiée ou cyclique, saturée ou insaturée, un sel de métal alcalin ou alcalino terreux, un ion ammonium,

ou

- un radical $NHR_4$, $R_4$ étant choisi parmi : un atome d'hydrogène, un radical phytyle ou benzyle, une chaîne alkyle en $C_1$-$C_{18}$, linéaire, ramifiée ou cyclique, saturée ou insaturée.

$R_1$ représente un radical choisi parmi: H, OH, alcoxy en $C_1$-$C_6$, préférentiellement méthoxy, une chaîne alkyle en $C_1$-$C_{18}$, linéaire, ramifiée ou cyclique, saturée ou insaturée ;
$R_2$ représente un radical choisi parmi: H, OH, alcoxy en $C_1$-$C_6$, préférentiellement méthoxy, et
b) au moins un polymère polyaminé choisi parmi :

(A) une polyalkylène polyamine ou un dérivé de polyalkylène polyamine choisi parmi :

(i) les polyalkylène polyamines ;
(ii) les dérivés alkylés de polyalkylène polyamines ;
(iii) les produits d'addition d'acides alkylcarboxyliques sur les polyalkylènepolyamines ;
(iv) les produits d'addition de cétones et d'aldéhydes sur les polyalkylènepolyamines ;
(v) les produits d'addition d'isocyanates et d'isothiocyanates sur les polyalkylènepolyamines ;
(vi) les produits d'addition d'oxyde d'alkylène ou de polymères blocs polyoxyde d'alkylène sur les polyalkylènepolyamines ;
(vii) les dérivés quaternisés de polyalkylène polyamines ;
(viii) les produits d'addition d'une silicone sur les polyalkylènepolyamines ;
(ix) un copolymère d'acide dicarboxylique et de polyalkylène polyamines ;

(B) les polyvinylimidazoles ;
(C) les polyvinylpyridines ;
(D) les produits d'addition de monomères 1-vinylimidazole de formule (1) :

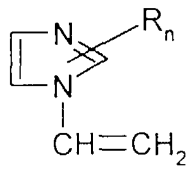

(1)

dans laquelle les radicaux R, identiques ou différents, représentent H, ou un radical alkyle en $C_1$-$C_6$, saturé ou insaturé, linéaire ou cyclique
n est un entier allant de 1 à 3,
avec les polyalkylènepolyamines (A)(i) à (A)(ix) ;
(E) les polymères à base d'acides aminés à chaîne latérale basique ;
(F) les dérivés réticulés des polymères (A)(i) à (A)(ix), (B) (C) (D) et (E).

**2.** Composition cosmétique et/ou dermatologique selon la revendication 1, caractérisée par le fait qu'elle comprend en outre
au moins un dérivé répondant à la formule (II) :

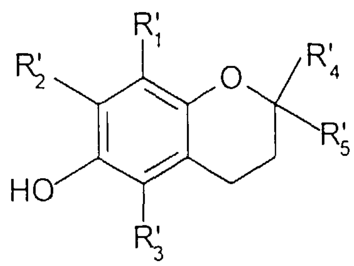

(II)

dans laquelle :

$R'_1$, $R'_2$, $R'_3$, identiques ou différents représentent un radical choisi parmi : H, OH, alkyle en $C_1$-$C_6$, préférentiellement méthyle,
$R'_4$ représente un radical choisi parmi : H, alkyle en $C_1$-$C_6$, préférentiellement méthyle,
$R'_5$ représente un radical choisi parmi : H, alkyle en $C_1$-$C_{18}$.

**3.** Composition cosmétique et/ou dermatologique selon l'une quelconque des revendications 1 et 2, caractérisée par le fait qu'elle comprend en outre au moins un dérivé de dibenzoylméthane répondant à la formule (III) suivante :

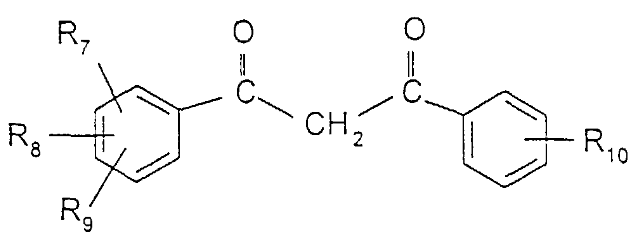

(III)

dans laquelle $R_7$, $R_8$, $R_9$ et $R_{10}$, identiques ou différents, représentent indépendamment l'hydrogène ou un radical hydroxyle ou un radical alkyle linéaire ou ramifié en $C_1$-$C_8$ ou un radical alcoxy linéaire ou ramifié en $C_1$-$C_8$.

**4.** Composition selon l'une quelconque des revendications précédentes, caractérisée par le fait que le polymère polyaminé est choisi parmi ceux comprenant au moins 5% de fonctions amines tertiaires, avantageusement au moins 10% de fonctions amines tertiaires, et encore plus préférentiellement au moins 20%.

**5.** Composition selon l'une quelconque des revendications précédentes, caractérisée par le fait que le polymère polyaminé est choisi parmi :

(A)

(i) les polyéthylèneimines hyperbranchées,
(ii) les dérivés alkylés de polyéthylèneimine ;
(iii) les produits d'addition d'acides alkylcarboxyliques sur la polyéthylèneimine ;
(iv) les produits d'addition de cétones et d'aldéhydes sur la polyéthylèneimine ;
(v) les produits d'addition d'isocyanates et de thioisocyanates sur la polyéthylèneimine ;
(vi) les produits d'addition d'oxyde d'alkylène ou de polymères blocs polyoxyde d'alkylène sur la polyéthylèneimine ;
(vii) les dérivés quaternisés de polyéthylèneimine ;
(viii) les produits d'addition d'une silicone sur la polyéthylèneimine ;
(ix) un copolymère d'acide dicarboxylique et de polyéthylèneimine ;

(B) les polyvinylimidazoles .

**6.** Composition selon l'une quelconque des revendications précédentes, caractérisée par le fait que le polymère polyaminé est choisi parmi les polyéthylèneimines hyperbranchées.

**7.** Composition selon l'une quelconque des revendications précédentes, caractérisée par le fait que le polymère polyaminé est présent dans la composition à une teneur allant de 0, 1 % à 10 % en poids, et de préférence de 0,25 % à 5 % en poids, par rapport au poids total de la composition

**8.** Composition selon l'une quelconque des revendications précédentes, caractérisée par le fait que le dérivé de l'acide cinnamique est le p-méthoxycinnamate de 2-éthylhexyle.

**9.** Composition selon l'une quelconque des revendications précédentes, caractérisée par le fait que le dérivé de l'acide cinnamique est présent dans la composition à une teneur allant de 0,1 % à 10 % en poids, par rapport au poids total de la composition, de préférence de 0,5 % à 5 % en poids, par rapport au poids total de la composition.

**10.** Composition selon l'une quelconque des revendications 2 à 9, caractérisée par le fait que le dérivé de formule (II) est un mélange de tocophérols naturels.

**11.** Composition selon l'une quelconque des revendications 2 à 10, caractérisée par le fait que le dérivé de formule (II) est présent dans la composition à une teneur allant de 0,5 % à 20 % en poids, par rapport au poids total de la composition, de préférence de 1 % à 10 % en poids, par rapport au poids total de la composition.

**12.** Composition selon l'une quelconque des revendications 3 à 11 précédentes, caractérisée par le fait que le dérivé du dibenzoylméthane de formule (III) est choisi parmi :

- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- le 4-tert.-butyl-4'-méthoxydibenzoylméthane,
- le 2-méthyl-5-isopropyl-4'-méthoxydibenzoylméthane,
- le 2-méthyl-5-tert.-butyl-4'-méthoxydibenzoylméthane,
- le 2,4-diméthyl-4'-méthoxydibenzoylméthane,
- le 2,6-diméthyl-4-tert.-butyl-4'-méthoxydibenzoylméthane,
- le 4,4'-diméthoxydibenzoylméthane.

**13.** Composition selon la revendication précédente, caractérisée par le fait que le composé de formule (III) est le 4-ter-butyl 4'-méthoxy dibenzoylméthane, répondant à la formule développée suivante :

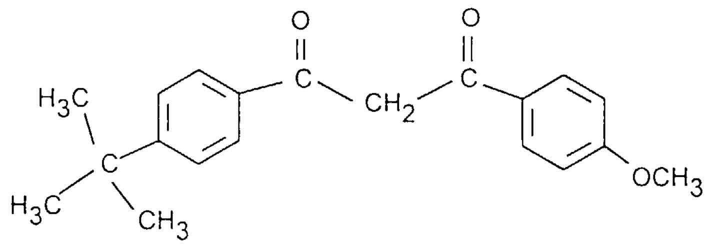

14. Composition selon la revendication précédente 12, caractérisée par le fait que le composé de formule (III) est le 4-isopropyl-dibenzoylméthane, répondant à la formule développée suivante :

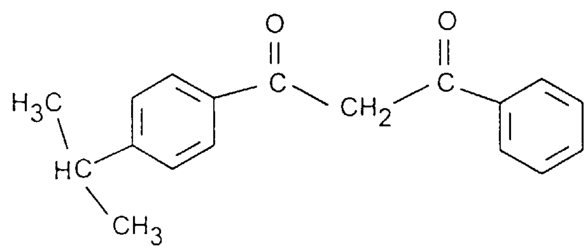

15. Composition selon l'une quelconque des revendications 3 à 14, caractérisée par le fait que le composé de formule (III) est présent dans la composition à une teneur allant de 0,2 % à 15 % en poids, par rapport au poids total de la composition, de préférence, de 0,2 % à 10 % en poids, par rapport au poids total de la composition.

16. Composition selon l'une quelconque des revendications précédentes, caractérisée par le fait qu'elle est sous la forme d'une émulsion huile-dans-eau.

17. Utilisation d'un polymère polyaminé tel que défini à l'une quelconque des revendications 1, 4 à 6 dans, ou pour la fabrication de, compositions cosmétiques et/ou dermatologiques contenant un dérivé de l'acide cinnamique tel que défini par la formule (II) à l'une quelconque des revendications 1 et 8 en vue d'améliorer dans ces compositions la stabilité au rayonnement UV du dérivé de l'acide cinnamique.

18. Procédé pour améliorer la stabilité au rayonnement UV des compositions cosmétiques et/ou dermatologiques comprenant un dérivé de l'acide cinnamique tel que défini à l'une quelconque des revendications 1 et 8, caractérisé par le fait qu'il consiste à introduire dans ces compositions une quantité efficace d'un polymère polyaminé tel que défini à l'une quelconque des revendications 1, 4 à 6.

19. Utilisation d'un polymère polyaminé tel que défini à l'une quelconque des revendications 1, 4 à 6 dans, ou pour la fabrication de, compositions cosmétiques et/ou dermatologiques contenant un dérivé de l'acide cinnamique tel que défini à l'une quelconque des revendications 1 et 8 en association avec au moins un composé tel que défini par la formule (II) à l'une quelconque des revendications 2 et 10 en vue d'améliorer dans ces compositions la stabilité au rayonnement UV du dérivé de l'acide cinnamique.

20. Procédé pour améliorer la stabilité au rayonnement UV des compositions cosmétiques et/ou dermatologiques comprenant un dérivé de l'acide cinnamique tel que défini à l'une quelconque des revendications 1 et 8, en association avec au moins un composé tel que défini par la formule (II) à l'une quelconque des revendications 2 et 10, caractérisé par le fait qu'il consiste à introduire dans ces compositions une quantité efficace d'un polymère polyaminé tel que défini à l'une quelconque des revendications 1, 4 à 6.

21. Utilisation d'un polymère polyaminé tel que défini à l'une quelconque des revendications 1, 4 à 6 dans, ou pour la fabrication de, compositions cosmétiques et/ou dermatologiques contenant un dérivé de l'acide cinnamique tel que défini par la formule (II) à l'une quelconque des revendications 1 et 8 en association avec au moins un dérivé de dibenzoylméthane tel que défini par la formule (III) à l'une quelconque des revendications 3, 12, 13 et 14 en vue d'améliorer dans ces compositions la stabilité au rayonnement UV du dérivé de l'acide cinnamique.

22. Procédé pour améliorer la stabilité au rayonnement UV des compositions cosmétiques et/ou dermatologiques

comprenant un dérivé de l'acide cinnamique tel que défini à l'une quelconque des revendications 1 et 8, en association avec au moins un dérivé de dibenzoylméthane tel que défini par la formule (III) à l'une quelconque des revendications 3, 12, 13 et 14, caractérisé par le fait qu'il consiste à introduire dans ces compositions une quantité efficace d'un polymère polyaminé tel que défini à l'une quelconque des revendications 1, 4 à 6.

**Patentansprüche**

**1.** Kosmetische und/oder dermatologische Zusammensetzung, die in einem kosmetisch und/oder dermatologisch akzeptablen Träger enthält:

a) mindestens ein Zimtsäurederivat, das der folgenden Formel (I) entspricht:

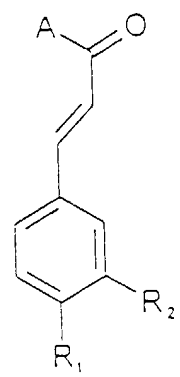

(I),

worin:

A bedeutet:

- eine Gruppe $OR_3$, wobei $R_3$ ausgewählt ist unter: Wasserstoff, Phythyl oder Benzyl, einer geradkettigen, verzweigten oder cyclischen, gesättigten oder ungesättigten $C_{1-18}$-Alkylgruppe, einem Alkali- oder Erdalkalimetall oder einem Ammoniumion; oder

  - eine Gruppe $NHR_4$, wobei $R_4$ ausgewählt ist unter: Wasserstoff, Phythyl oder Benzyl, oder einer geradkettigen, verzweigten oder cyclischen, gesättigten oder ungesättigten $C_{1-18}$-Alkylgruppe;

$R_1$ ausgewählt ist unter: H, OH, $C_{1-6}$-Alkoxy, vorzugsweise Methoxy, oder einer geradkettigen, verzweigten oder cyclischen, gesättigten oder ungesättigten $C_{1-18}$-Alkylgruppe; und
$R_2$ ausgewählt ist unter: H, OH oder $C_{1-6}$-Alkoxy, vorzugsweise Methoxy; und
b) mindestens ein Polyaminpolymer, das unter den folgenden Gruppen ausgewählt ist:

(A) den Polyalkylenpolyaminen oder Polyalkylenpolyaminderivaten, die ausgewählt sind unter:

(i) den Polyalkylenpolyaminen;
(ii) den alkylierten Derivaten von Polyalkylenpolyaminen;
(iii) den Additionsprodukten von Alkylcarbonsäuren und Polyalkylenpolyaminen;
(iv) den Additionsprodukten von Ketonen und Aldehyden und Polyalkylenpolyaminen;
(v) den Additionsprodukten von Isocyanaten und Isothiocyanaten und Polyalkylenpolyaminen;
(vi) den Additionsprodukten von Alkylenoxid oder Blockpolymeren von Polyalkylenoxid mit Polyalkylenpolyaminen;
(vii) den quaternisierten Derivaten von Polyalkylenpolyaminen;
(viii) den Additionsprodukten von Siliconen und Polyalkylenpolyaminen;
(ix) den Copolymeren von Dicarbonsäuren und Polyalkylenpolyaminen;

(B) den Polyvinylimidazolen;

(C) den Polyvinylpyridinen;
(D) den Additionsprodukten von
1-Vinylimidazolmonomeren der Formel (1):

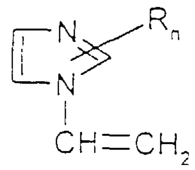

(1),

worin die Gruppen R, die identisch oder voneinander verschieden sind, H oder eine geradkettige oder cyclische, gesättigte oder ungesättigte $C_{1-6}$-Alkylgruppe und n eine ganze Zahl von 1 bis 3 bedeuten, mit den Polyalkylenpolyaminen (A)(i) bis (A)(ix);
(E) den Polymeren auf der Basis von Aminosäuren mit basischer Seitenkette;
(F) den vernetzten Derivaten von Polymeren (A)(i) bis (A)(ix), (B), (C), (D) und (E).

**2.** Kosmetische und/oder dermatologische Zusammensetzung nach Anspruch 1, dadurch gekennzeichnet, daß sie ferner mindestens ein Derivat der folgenden Formeln (II) enthält:

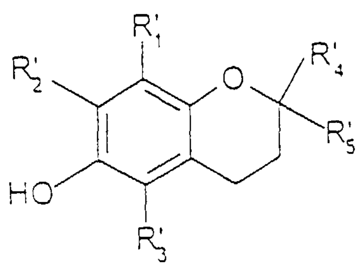

(II),

worin:

die Gruppen $R'_1$, $R'_2$ und $R'_3$, die identisch oder voneinander verschieden sind, ausgewählt sind unter: H, OH, $C_{1-6}$-Alkyl, vorzugsweise Methyl;
die Gruppe $R'_4$ ausgewählt ist unter: H, $C_{1-6}$-Alkyl, vorzugsweise Methyl;
die Gruppe $R'_5$ ausgewählt ist unter: H, $C_{1-18}$-Alkyl.

**3.** Kosmetische und/oder dermatologische Zusammensetzung nach einem der Ansprüche 1 oder 2, dadurch gekennzeichnet, daß sie ferner mindestens ein Dibenzoylmethanderivat der folgenden Formel (III) enthält:

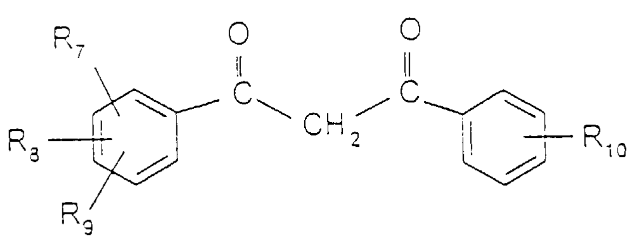

(III),

worin die Gruppen $R_7$, $R_8$, $R_9$ und $R_{10}$, die identisch oder voneinander verschieden sind, unabhängig voneinander Wasserstoff, Hydroxy, eine geradkettige oder verzweigte $C_{1-8}$-Alkylgruppe oder eine geradkettige oder verzweigte $C_{1-8}$-Alkoxygruppe bedeuten.

4. Zusammensetzung nach einem der vorhergehenden Ansprüche, dadurch gekennzeichnet, daß das Polyaminpolymer unter den Polymeren ausgewählt ist, die mindestens 5 % tertiäre Aminogruppen enthalten, vorteilhaft mindestens 10 % tertiäre Aminogruppen und noch bevorzugter mindestens 20 %.

5. Zusammensetzung nach einem der vorhergehenden Ansprüche, dadurch gekennzeichnet, daß das Polyaminpolymer ausgewählt ist unter:

(A)

(i) den hochverzweigten Polyethyleniminen;
(ii) den alkylierten Polyethyleniminderivaten;
(iii) den Additionsprodukten von Alkylcarbonsäuren und Polyethylenimin;
(iv) den Additionsprodukten von Ketonen und Aldehyden und Polyethylenimin;
(v) den Additionsprodukten von Isocyanaten und Isothiocyanaten und Polyethylenimin;
(vi) den Additionsprodukten von Alkylenoxid oder Blockpolymeren von Polyalkylenoxid und Polyethylenimin;
(vii) den quaternisierten Derivaten von Polyethylenimin;
(viii) den Additionsprodukten von Siliconen und Polyethylenimin;
(ix) den Copolymeren von Dicarbonsäuren und Polyethylenimin;

(B) den Polyvinylimidazolen.

6. Zusammensetzung nach einem der vorhergehenden Ansprüche, dadurch gekennzeichnet, daß das Polyaminpolymer unter den hochverzweigten Polyethyleniminen ausgewählt ist.

7. Zusammensetzung nach einem der vorhergehenden Ansprüche, dadurch gekennzeichnet, daß das Polyaminpolymer in der Zusammensetzung in Mengenanteilen im Bereich von 0,1 bis 10 Gew.-% und vorzugsweise 0,25 bis 5 Gew.-%, bezogen auf das Gesamtgewicht der Zusammensetzung, vorliegt.

8. Zusammensetzung nach einem der vorhergehenden Ansprüche, dadurch gekennzeichnet, daß das Zimtsäurederivat das 2-Ethylhexyl-p-methoxycinnamat ist.

9. Zusammensetzung nach einem der vorhergehenden Ansprüche, dadurch gekennzeichnet, daß das Zimtsäurederivat in der Zusammensetzung in Mengenanteilen im Bereich von 0,1 bis 10 Gew.-% und vorzugsweise 0,5 bis 5 Gew.-%, bezogen auf das Gesamtgewicht der Zusammensetzung, vorliegt.

10. Zusammensetzung nach einem der Ansprüche 2 bis 9, dadurch gekennzeichnet, daß das das Derivat der Formel (II) ein Gemisch von natürlichen Tocopherolen ist.

11. Zusammensetzung nach einem der Ansprüche 2 bis 10, dadurch gekennzeichnet, daß das Derivat der Formel (II) in der Zusammensetzung in Mengenanteilen im Bereich von 0,5 bis 20 Gew.-% und vorzugsweise 1 bis 10 Gew.-%, bezogen auf das Gesamtgewicht der Zusammensetzung, vorliegt.

12. Zusammensetzung nach einem der Ansprüche 3 bis 11, dadurch gekennzeichnet, daß das Dibenzoylmethanderivat ausgewählt ist unter:

- 2-Methyl-dibenzoylmethan,
- 4-Methyl-dibenzoylmethen,
- 4-Isoproyl-dibenzoylmethan,
- 4-*tert*.-Butyl-dibenzoylmethan,
- 2,4-Dimethyl-dibenzoylmethan,
- 2,5-Dimethyl-dibenzoylmethan,
- 4,4'-Diisopropyl-dibenzoylmethan,
- 4-*tert*.-Butyl-4'-methoxy-dibenzoylmethan,
- 2-Methyl-5-isopropyl-4'-methoxy-dibenzoylmethan,
- 2-Methyl-5-*tert*.-butyl-4'-methoxy-dibenzoylmethan,
- 2,4-Dimethyl-4'-methoxy-dibenzoylmethan,
- 2,6-Dimethyl-4-*tert*.-butyl-4'-methoxy-dibenzoylmethan, und

- 4,4'-Dimethoxy-dibenzoylmethan.

**13.** Zusammensetzung nach dem vorhergehenden Anspruch, dadurch gekennzeichnet, daß die Verbindung der Formel (III) das 4-*tert*.-Butyl-4'-methoxydibenzoyl-methan der folgenden Strukturformel ist:

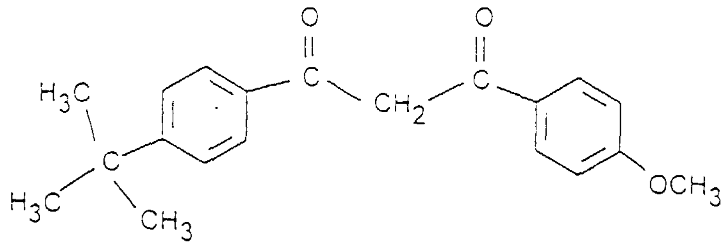

**14.** Zusammensetzung nach dem vorhergehenden Anspruch 12, dadurch gekennzeichnet, daß die Verbindung der Formel (III) das 4-Isopropyl-dibenzoyl-methan der folgenden Strukturformel ist:

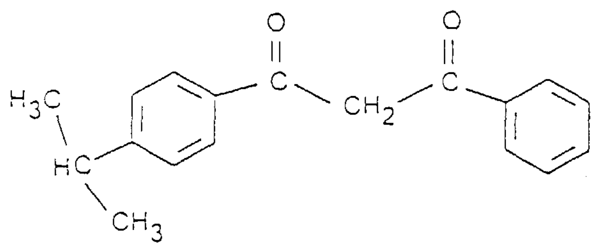

**15.** Zusammensetzung nach einem der Ansprüche 3 bis 14, dadurch gekennzeichnet, daß die Verbindung der Formel (III) in der Zusammensetzung in einem Mengenanteil von 0,2 bis 15 Gew.-%, bezogen auf das Gesamtgewicht der Zusammensetzung, und vorzugsweise 0,2 bis 10 Gew.-%, bezogen auf das Gesamtgewicht der Zusammensetzung, vorliegt.

**16.** Zusammensetzung nach einem der vorhergehenden Ansprüche, dadurch gekennzeichnet, daß sie in Form einer Öl-in-Wasser-Emulsion vorliegt.

**17.** Verwendung eines Polyaminpolymers nach einem der Ansprüche 1, 4 bis 6 in kosmetischen und/oder dermatologischen Zusammensetzungen, die ein Zimtsäurederivat der Formel (I) nach einem der Ansprüche 1 bis 8 enthalten, oder seine Verwendung zur Herstellung dieser Zusammensetzungen zur Verbesserung der Stabilität des Zimtsäurederivats gegenüber UV-Strahlung in diesen Zusammensetzungen.

**18.** Verfahren zur Verbesserung der Stabilität von kosmetischen und/oder dermatologischen Zusammensetzungen, die ein Zimtsäurederivat nach einem der Ansprüche 1 bis 8 enthalten, gegenüber UV-Strahlung, dadurch gekennzeichnet, daß in die Zusammensetzung eine wirksame Menge eines Polyaminpolymers nach einem der Ansprüche 1, 4 bis 6 eingearbeitet wird.

**19.** Verwendung eines Polyaminpolymers nach einem der Ansprüche 1, 4 bis 6 in kosmetischen und/oder dermatologischen Zusammensetzungen, die ein Zimtsäurederivat nach einem der Ansprüche 1 bis 8 in Kombination mit mindestens einer Verbindung der Formel (II) nach einem der Ansprüche 2 bis 10 enthalten, oder seine Verwendung zur Herstellung dieser Zusammensetzungen zur Verbesserung der Stabilität des Zimtsäurederivats gegenüber UV-Strahlung in diesen Zusammensetzungen.

**20.** Verfahren zur Verbesserung der Stabilität von kosmetischen und/oder dermatologischen Zusammensetzungen, die ein Zimtsäurederivat nach einem der Ansprüche 1 bis 8 in Kombination mit mindestens einer Verbindung der Formel (II) nach einem der Ansprüche 2 bis 10 enthalten, das dadurch gekennzeichnet ist, daß es darin besteht, in die Zusammensetzung eine wirksame Menge eines Polyaminpolymers nach einem der Ansprüche 1, 4 bis 6 einzuarbeiten.

**21.** Verwendung eines Polyaminpolymers nach einem der Ansprüche 1, 4 bis 6 in kosmetischen und/oder dermatologischen Zusammensetzungen, die ein Zimtsäurederivat nach einem der Ansprüche 1 bis 8 in Kombination mit mindestens einem Dibenzoylmethanderivat der Formel (III) nach einem der Ansprüche 3, 12, 13 und 14 enthalten, oder seine Verwendung zur Herstellung dieser Zusammensetzungen zur Verbesserung der Stabilität des Zimtsäurederivats gegenüber UV-Strahlung in diesen Zusammensetzungen.

**22.** Verfahren zur Verbesserung der Stabilität von kosmetischen und/oder dermatologischen Zusammensetzungen, die ein Zimtsäurederivat nach einem der Ansprüche 1 bis 8 in Kombination mit mindestens einem Dibenzoylmethanderivat der Formel (III) nach einem der Ansprüche 3, 12, 13 und 14 enthalten. das dadurch gekennzeichnet ist, daß es darin besteht, in die Zusammensetzungen eine wirksame Menge eines Polyaminpolymers nach einem der Ansprüche 1, 4 bis 6 einzuarbeiten.

## Claims

**1.** Cosmetic and/or dermatological composition comprising, in a cosmetically and/or dermatologically acceptable vehicle:

a) at least one cinnamic acid derivative corresponding to the following formula (I):

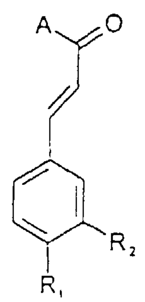

(I)

in which:
A represents:

- an $OR_3$ radical, $R_3$ being chosen from: a hydrogen atom, a phytyl or benzyl radical, a saturated or unsaturated, linear, branched or cyclic, $C_1$-$C_{18}$ alkyl chain, an alkali or alkaline earth metal salt or an ammonium ion, or
- an $NHR_4$ radical, $R_4$ being chosen from: a hydrogen atom, a phytyl or benzyl radical or a saturated or unsaturated, linear, branched or cyclic, $C_1$-$C_{18}$ alkyl chain,

$R_1$ represents a radical chosen from: H, OH, $C_1$-$C_6$ alkoxy, preferably methoxy, or a saturated or unsaturated, linear, branched or cyclic, $C_1$-$C_{18}$ alkyl chain;
$R_2$ represents a radical chosen from: H, OH or $C_1$-$C_6$ alkoxy, preferably methoxy,

and

b) at least one polyamino polymer chosen from:

(A) a polyalkylene polyamine or a polyalkylene polyamine derivative chosen from:

(i) polyalkylene polyamines;
(ii) alkylated derivatives of polyalkylene polyamines;
(iii) addition products of alkylcarboxylic acids with polyalkylene polyamines;
(iv) addition products of ketones and aldehydes with polyalkylene polyamines;
(v) addition products of isocyanates and isothiocyanates with polyalkylene polyamines;
(vi) addition products of alkylene oxide or of poly(alkylene oxide) block polymers with polyalkylene

polyamines;
(vii) quaternized derivatives of polyalkylene polyamines;
(viii) addition products of a silicone with polyalkylene polyamines;
(ix) a copolymer of dicarboxylic acid and of polyalkylene polyamines;

(B) polyvinylimidazoles;
(C) polyvinylpyridines;
(D) addition products of 1-vinylimidazole monomers of formula (1):

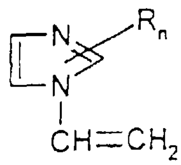

(1)

in which the R radicals, which are identical or different, represent H or a saturated or unsaturated, linear or cyclic, $C_1$-$C_6$ alkyl radical n is an integer ranging from 1 to 3,
with the polyalkylene polyamines (A)(i) to (A)(ix);
(E) polymers based on amino acids containing a basic side chain;
(F) crosslinked derivatives of the polymers (A) (i) to (A) (ix) , (B), (C), (D) and (E) .

**2.** Cosmetic and/or dermatological composition according to Claim 1, characterized in that it additionally comprises at least one derivative corresponding to the formula (II):

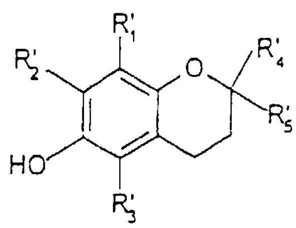

(II)

in which:

R'$_1$, R'$_2$ and R'$_3$, which are identical or different, represent a radical chosen from: H, OH or $C_1$-$C_6$ alkyl, preferably methyl,
R'$_4$ represents a radical chosen from: H or $C_1$-$C_6$ alkyl, preferably methyl,
R'$_5$ represents a radical chosen from: H or $C_1$-$C_{18}$ alkyl.

**3.** Cosmetic and/or dermatological composition according to either one of Claims 1 and 2, characterized in that it additionally comprises at least one dibenzoylmethane derivative corresponding to the following formula (III):

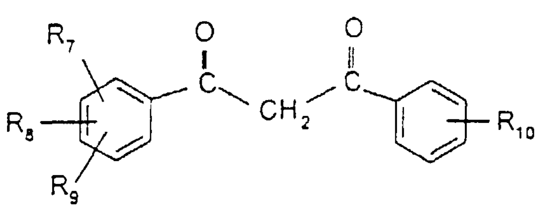

(III)

in which $R_7$, $R_8$, $R_9$ and $R_{10}$, which are identical or different, independently represent hydrogen or a hydroxyl radical or a linear or branched $C_1$-$C_8$ alkyl radical or a linear or branched $C_1$-$C_8$ alkoxy radical.

**4.** Composition according to any one of the preceding claims, characterized in that the polyamino polymer is chosen from those comprising at least 5% of tertiary amine functional groups, advantageously at least 10% of tertiary amine functional groups and more preferably still at least 20%.

**5.** Composition according to any one of the preceding claims, characterized in that the polyamino polymer is chosen from:

(A)

(i) hyperbranched polyethyleneimines,
(ii) alkylated polyethyleneimine derivatives;
(iii) addition products of alkylcarboxylic acids with polyethyleneimine;
(iv) addition products of ketones and aldehydes with polyethyleneimine;
(v) addition products of isocyanates and isothiocyanates with polyethyleneimine;
(vi) addition products of alkylene oxide or of poly(alkylene oxide) block polymers with polyethyleneimine;
(vii) quaternized derivatives of polyethyleneimine;
(viii) addition products of a silicone with polyethyleneimine;
(ix) a copolymer of dicarboxylic acid and of polyethyleneimine;

(B) polyvinylimidazoles.

**6.** Composition according to any one of the preceding claims, characterized in that the polyamino polymer is chosen from hyperbranched polyethyleneimines.

**7.** Composition according to any one of the preceding claims, characterized in that the polyamino polymer is present in the composition at a content ranging from 0.1% to 10% by weight and preferably from 0.25% to 5% by weight, with respect to the total weight of the composition.

**8.** Composition according to any one of the preceding claims, characterized in that the cinnamic acid derivative is 2-ethylhexyl p-methoxycinnamate.

**9.** Composition according to any one of the preceding claims, characterized in that the cinnamic acid derivative is present in the composition at a content ranging from 0.1% to 10% by weight with respect to the total weight of the composition, preferably from 0.5% to 5% by weight with respect to the total weight of the composition.

**10.** Composition according to any one of Claims 2 to 9, characterized in that the derivative of formula (II) is a mixture of natural tocopherols.

**11.** Composition according to any one of Claims 2 to 10, characterized in that the derivative of formula (II) is present in the composition at a content ranging from 0.5% to 20% by weight with respect to the total weight of the composition, preferably from 1% to 10% by weight with respect to the total weight of the composition.

**12.** Composition according to any one of the preceding Claims 3 to 11, characterized in that the dibenzoylmethane derivative of formula (III) is chosen from:

- 2-methyldibenzoylmethane,
- 4-methyldibenzoylmethane,
- 4-isopropyldibenzoylmethane,
- 4-tert-butyldibenzoylmethane,
- 2,4-dimethyldibenzoylmethane,
- 2,5-dimethyldibenzoylmethane,
- 4,4'-diisopropyldibenzoylmethane,
- 4-tert-butyl-4'-methoxydibenzoylmethane,
- 2-methyl-5-isopropyl-4'-methoxydibenzoylmethane,
- 2-methyl-5-tert-butyl-4'methoxydibenzoylmethane,
- 2,4-dimethyl-4'-methoxydibenzoylmethane,
- 2,6-dimethyl-4-tert-butyl-4'-methoxydibenzoylmethane,
- 4,4'-dimethoxydibenzoylmethane.

**13.** Composition according to the preceding claim, characterized in that the compound of formula (III) is 4-tert-butyl-4'-methoxydibenzoyl-methane corresponding to the following expanded formula:

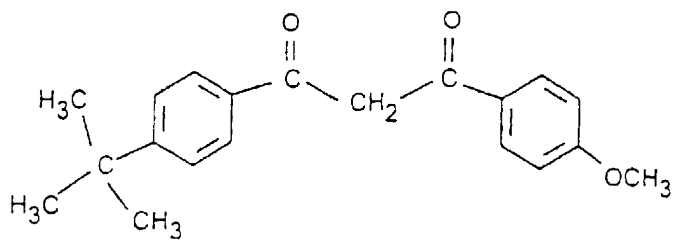

**14.** Composition according to the preceding Claim 12, characterized in that the compound of formula (III) is 4-isopropyldibenzoylmethane corresponding to the following expanded formula:

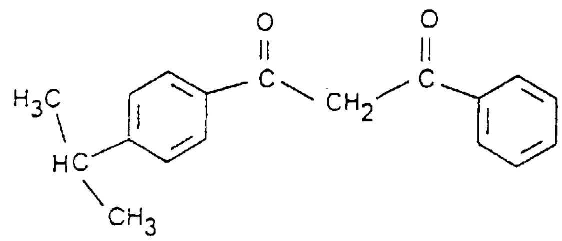

**15.** Composition according to any one of Claims 3 to 14, characterized in that the compound of formula (III) is present in the composition at a content ranging from 0.2% to 15% by weight with respect to the total weight of the composition, preferably from 0.2% to 10% by weight with respect to the total weight of the composition.

**16.** Composition according to any one of the preceding claims, characterized in that it is in the form of an oil-in-water emulsion.

**17.** Use of a polyamino polymer as defined in any one of Claims 1, 4 to 6 in, or for the manufacture of, cosmetic and/or dermatological compositions containing a cinnamic acid derivative as defined by the formula (I) in either one of Claims 1 and 8, for the purpose of improving, in these compositions, the stability to UV radiation of the cinnamic acid derivative.

**18.** Process for improving the stability to UV radiation of cosmetic and/or dermatological compositions comprising a cinnamic acid derivative as defined in either one of Claims 1 and 8, characterized in that it consists in introducing, into these compositions, an effective amount of a polyamino polymer as defined in any one of Claims 1, 4 to 6.

**19.** Use of a polyamino polymer as defined in any one of Claims 1, 4 to 6 in, or for the manufacture of, cosmetic and/or dermatological compositions containing a cinnamic acid derivative as defined in either one of Claims 1 and 8 in combination with at least one compound as defined by the formula (II) in either one of Claims 2 and 10, for the purpose of improving, in these compositions, the stability to UV radiation of the cinnamic acid derivative.

**20.** Process for improving the stability to UV radiation of cosmetic and/or dermatological compositions comprising a cinnamic acid derivative as defined in either one of Claims 1 and 8 in combination with at least one compound as defined by the formula (II) in either one of Claims 2 and 10, characterized in that it consists in introducing, into these compositions, an effective amount of a polyamino polymer as defined in any one of Claims 1, 4 to 6.

**21.** Use of a polyamino polymer as defined in any one of Claims 1, 4 to 6 in, or for the manufacture of, cosmetic and/or dermatological compositions containing a cinnamic acid derivative as defined by the formula (I) in either one of Claims 1 and 8 in combination with at least one dibenzoylmethane derivative as defined by the formula (III) in any one of Claims 3, 12, 13 and 14, for the purpose of improving, in these compositions, the stability to UV radiation of the cinnamic acid derivative.

**22.** Process for improving the stability to UV radiation of cosmetic and/or dermatological compositions comprising a cinnamic acid derivative as defined in either one of Claims 1 and 8 in combination with at least one dibenzoylmethane derivative as defined by the formula (III) in any one of Claims 3, 12, 13 and 14, characterized in that it consists in introducing, into these compositions, an effective amount of a polyamino polymer as defined in any

one of Claims 1, 4 to 6.